# EUROPEAN PATENT SPECIFICATION

(11) **EP 4 215 107 B1**
(45) Date of publication and mention of the grant of the patent: **04.11.2026**
(21) Application number: 21869432.1
(22) Date of filing: 16.09.2021
(51) Int. Cl.: G01L 11/02, G01L 1/24, A61B 5/0215, A61B 5/03

(54) **IN VIVO PRESSURE MEASUREMENT DEVICE**
VORRICHTUNG ZUR DRUCKMESSUNG IN VIVO
DISPOSITIF DE MESURE DE LA PRESSION IN VIVO

(30) Priority: 16.09.2020 JP 2020155186
(43) Date of publication of application: 26.07.2023
(73) Proprietor: Furukawa Electric Co., Ltd., Tokyo 100-8322 (JP)
(72) Inventor: HASEGAWA, Hideaki, Tokyo 100-8322 (JP); TAMAOKA, Hiroyuki, Tokyo 100-8322 (JP); FUJIHATA, Takashi, Tokyo 100-8246 (JP)
(74) Representative: SSM Sandmair
(86) International application number: PCT/JP2021/034182
(87) International publication number: WO 2022/059749

(56) References cited:
- EP-A1- 3 940 354
- JP-A- 2015 517 678
- JP-A- 2016 525 908
- JP-A- 2018 532 987
- US-A- 4 915 473
- US-A1- 2018 228 385
- US-A1- 2019 159 688
- US-B1- 6 677 576

## Description

### Field

The present invention relates to an in vivo pressure measurement device.

### Background

Conventionally, an invasive blood pressure monitor as disclosed in Patent Literature 1 and a non-invasive blood pressure monitor as disclosed in Patent literature 2 are known.

### Citation List

### Patent Literature

Patent Literature 1: Japanese Patent Application Laid-open No. 2018-187146
Patent Literature 2: Japanese Patent Application Laid-open No. 2020-028478

EP 3 940 354 A1 discloses a pressure sensor including a light source that outputs signal light, a sensor optical fiber where the signal light is input and the signal light is propagated with a loss of 0.3 dB/m or more, and an optical receiver that receives the signal light propagated through the sensor optical fiber are included, and pressure applied to the sensor optical fiber is detected on a basis of intensity of the signal light received by the optical receiver. An optical transmission body including the sensor optical fiber and a transmission optical fiber connected to the sensor optical fiber and smaller in propagation loss than the sensor optical fiber may be included.

US 4,915,473 A discloses a pressure sensing device utilizing a polymer optical fiber with a core comprised of a flexible thermoplastic aliphatic segmented polyurethane. The use of an optical fiber based pressure sensor eliminates the electromagnetic interference problems associated with conventional electronic pressure sensors. Use of polyurethane as the core material for the optical fiber results in an optical fiber with high flexibility, thus making the inventive pressure sensing device more rugged and durable than other optical fiber based pressure sensors.

US 6,677,576 B1 discloses sensors and sensing apparatuses which are based on fused tapered fiber optic couplers with the taper waist portion formed into a loop or cleaved to provide a reflecting end. Light is typically input to the taper waist portion along a single input fiber, and bending of the loop or cleaved taper waist portion causes changes in the coupling ratio between the output fibers. The taper waist portion may be formed into a loop with a small bend radius without loss, and sensors embodying the claimed invention are particularly suitable for in-vivo measurement of pressure, the taper waist portions advantageously being arranged in contact with a membrane which deflects according to surrounding pressure. Further embodiments are described which are suitable for measurement of fluid flow velocity and/or acceleration.

US 2019/159688 A1 discloses a device for determining an arterial compliance of a subject. The device comprises an inflatable cuff, a pressure sensor which is configured to sense a pressure signal that is indicative of a pressure within the cuff, a second sensor which is at least partly integrated in the cuff and configured to sense a second signal that is responsive to expansions and contractions of the cuff caused by a pulsating blood flow in an artery of the subject, and a processing unit. The processing unit is configured to determine based on said part of the pressure signal a pulse pressure of the subject, to determine based on said part of the second signal an arterial volume change of the artery of the subject during at least one cardiac cycle, and to determine the arterial compliance of the subject based on pulse pressure and the arterial volume change.
US 4 611 600 A discloses an optical blood pressure catheter which includes an elongated flexible light conductor for transmitting light received at its proximal end to its distal end and for transmitting light received at the distal end back to the proximal end. A pressure transducer is located adjacent the distal end and includes a light reflector which is spaced along a light path from the distal end and is fixed in position relative thereto so as to receive and reflect light emitted from the distal end back into the distal end. A pressure responsive membrane is located intermediate the distal end and the reflector and is movable transversely of the light path in dependence upon the pressure exerted on the exterior surface of the membrane. A shutter carried by the membrane moves therewith transversely of the light path for blocking light along the light path so that the light received by the distal end is attenuated in dependence upon the pressure exerted on the membrane.
US 2015/141854 A1 discloses an apparatus including an elongated assembly, at least a portion of which is sized, shaped, or otherwise configured to be inserted into a human body to measure a physiological parameter at an internal location within the body. The elongated assembly includes an elongated member having a first length and an outer surface, a coil disposed about at least a portion of the elongated member, the coil having a second length, and at least one standoff member positioned between the outer surface of the elongated member and the coil, where the at least one member is configured to prevent the coil from contacting an optical fiber positioned between the elongated member and the coil.

### Summary

### Technical Problem

In both types of blood pressure monitors, it would be beneficial if the size thereof can be made smaller and the structure thereof can be more simplified, for example.

In addition to blood pressure, it would be even more beneficial if pressure and forces acting in a living body can be measured as well.

It is therefore an object of the present invention, for example, to acquire a more improved and new in vivo pressure measurement device.

### Solution to Problem

The invention is defined by the subject-matter of claim 1. The dependent claims relate to preferred embodiments of the invention.

An in vivo pressure measurement device according
to the present invention includes: a light source configured to output test light; an optical fiber, to which the test light is input, at least partially including a sensor optical fiber configured to transmit the test light with a loss of 0.3 dB/m or more; and a light receiving unit configured to receive the test light transmitted through the sensor optical fiber, wherein the in vivo pressure measurement device is configured to measure pressure in a living body acting on the sensor optical fiber based on intensity of the test light received at the light receiving unit.

In the in vivo pressure measurement device, the pressure in the living body may pressure of a fluid in the living body.

In the in vivo pressure measurement device, the sensor optical fiber may be a plastic fiber.

In the in vivo pressure measurement device, the sensor optical fiber may include a curved part.

In the in vivo pressure measurement device, the sensor optical fiber may configured to be inserted into the living body.

In the in vivo pressure measurement device, the sensor optical fiber may be configured to measure pressure of an ex vivo fluid to which pressure of an in vivo fluid is propagated.

The in vivo pressure measurement device may include a flexible support member configured to extend along the optical fiber and support the optical fiber.

The in vivo pressure measurement device may include a tubular member configured to house the optical fiber.

In the in vivo pressure measurement device, the tubular member may be flexible.

The in vivo pressure measurement device may include a cover configured to cover the sensor optical fiber in a state capable of introducing an in vivo fluid or an ex vivo fluid to which pressure of the in vivo fluid is propagated.

In the in vivo pressure measurement device, the sensor optical fiber may be located outside the living body, and configured to measure pressure of an in vivo fluid indirectly acting on the sensor optical fiber via the living body.

In the in vivo pressure measurement device, the sensor optical fiber may be configured to be disposed between the living body and a cuff located outside the living body, the cuff being configured to constrict a blood vessel in the living body by internal air pressure and change the air pressure.

In the in vivo pressure measurement device, the optical fiber may include: the sensor optical fiber; and a delivery optical fiber connected to the sensor optical fiber and having a lower transmission loss than the sensor optical fiber does.

In the in vivo pressure measurement device, in wavelengths of the test light, the sensor optical fiber may be a single-mode optical fiber, and the delivery optical fiber between the sensor optical fiber and the light source may be a multi-mode optical fiber.

In the in vivo pressure measurement device, the sensor optical fiber and the delivery optical fiber may be fusion-spliced.

In the in vivo pressure measurement device, the light receiving unit may be configured to receive the test light input from one end of the optical fiber and output from another end of the optical fiber.

In the in vivo pressure measurement device, the sensor optical fiber includes a core, a cladding surrounding the core, and a plurality of nanostructures near an interface between the core and the cladding, and the nanostructure may have a cross-sectional diameter of 100 nm or less in a cross section perpendicular to a longitudinal direction of the sensor optical fiber.

In the in vivo pressure measurement device, the nanostructure may be a microparticle, a tube or a void.

In the in vivo pressure measurement device, an outer periphery of the sensor optical fiber may include an uneven structure of 5 nm to 100 nm.

The in vivo pressure measurement device may include an arithmetic processing unit configured to acquire the pressure acting on the sensor optical fiber based on the intensity of the test light.

Moreover, an in vivo pressure measurement device according to the present invention includes, for example: a light source configured to output test light; an optical fiber, to which the test light is input, including a sensor part from which light leaks due to an applied external force; and a light receiving unit configured to receive the test light transmitted through the sensor part, wherein the in vivo pressure measurement device is configured to measure pressure in a living body acting on the sensor part based on intensity of the test light received at the light receiving unit.

In the in vivo pressure measurement device, a transmission loss in the sensor part may increase as the external force acting on the sensor part increases.

### Advantageous Effects of Invention

According to the present invention, for example, it is possible to acquire an improved and new in vivo
pressure measurement device.

### Brief Description of Drawings

FIG. 1 is an exemplary schematic diagram of an in vivo pressure measurement device according to a first embodiment.
FIG. 2 is an exemplary schematic diagram of an optical fiber according to the embodiment.
FIG. 3 is an exemplary and schematic cross-sectional view taken along the longitudinal direction of a part of a sensor part according to the embodiment.
FIG. 4 is an exemplary and schematic cross-sectional view perpendicular to the longitudinal direction of the sensor part according to the embodiment.
FIG. 5 is a graph presenting an example of change over time of detection values of pressure acquired by the in vivo pressure measurement device of the embodiment and a conventional pressure sensor.
FIG. 6 is an exemplary schematic diagram of an in vivo pressure measurement device according to a second embodiment.
FIG. 7 is an exemplary schematic diagram of an in vivo pressure measurement device according to a third embodiment.
FIG. 8 is an exemplary schematic diagram of an in vivo pressure measurement device according to a modification of the third embodiment.
FIG. 9 is an exemplary schematic diagram of an in vivo pressure measurement device according to a fourth embodiment.
FIG. 10 is an exemplary and schematic diagram of a part of the in vivo pressure measurement device according to the fourth embodiment.
FIG. 11 is an exemplary schematic diagram of an in vivo pressure measurement device according to a fifth embodiment, which illustrates a state where no pressure is acting on the sensor part from a living body.
FIG. 12 is an exemplary schematic diagram of the in vivo pressure measurement device according to the fifth embodiment, which illustrates a state where pressure is acting on the sensor part from the living body.
FIG. 13 is an exemplary and schematic cross-sectional view perpendicular to the longitudinal direction of the sensor part according to a modification of the embodiment. Description of Embodiments

Hereinafter, exemplary embodiments and modifications of the present invention will be disclosed. The configurations of the embodiments and modifications presented below as well as the actions and results (effects) brought about by such configurations are examples. The present invention can also be achieved by configurations other than those disclosed in the following embodiments and modifications. In addition, according to the present invention, it is possible to acquire at least one of the various kinds of effects (including derivative effects) achieved by the configurations.

The embodiments and modifications described hereinafter have similar constituent elements. Therefore, according to the configuration of each of the embodiments and modifications, it is possible to acquire similar actions and effects based on the similar constituent elements. Furthermore, hereinafter, similar reference signs may be applied to the similar constituent elements, and duplicated explanations may be omitted.

In the present descriptions, ordinal numbers are assigned for convenience in order to distinguish components, members, parts, and the like, and do not indicate priority or order thereof.

### [First Embodiment]

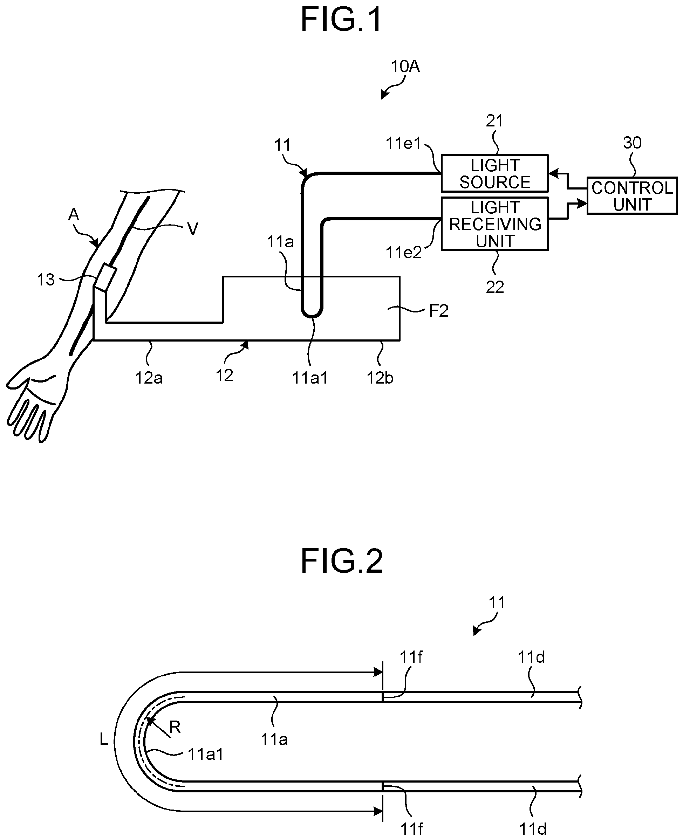

FIG. 1 is a schematic diagram of a pressure measurement device 10A according to a first embodiment. In the present embodiment, the pressure measurement device 10A is a device that measures blood pressure as fluid pressure in a living body, which is an invasive blood pressure measurement device. Note that blood is an example of a fluid in a living body A as a target of pressure measurement, and may also be referred to as a first fluid. The pressure measurement device 10A is an example of an in vivo pressure measurement device.

As illustrated in FIG. 1, the pressure measurement device 10A includes an optical fiber 11, a tube set 12, a catheter 13, a light source 21, a light receiving unit 22, and a control unit 30.

An end part 11e1 in the longitudinal direction of the optical fiber 11 is optically connected to the light source 21, and an end part 11e2 in the longitudinal direction of the optical fiber 11 is optically connected to the light receiving unit 22. Test light emitted from the light source 21 is input into the optical fiber 11 from the end part 11e1, transmitted through the optical fiber 11 including a sensor part 11a, output outside the optical fiber 11 from the end part 11e2, and received at the light receiving unit 22. The end part 11e1 is an example of one end, and the end part 11e2 is an example of the other end.

The light source 21 has, for example, a laser diode that outputs light with a wavelength of 400 [nm] or more and 500 [nm] or less, for example. The light source 21 may also output optical pulses intermittently at prescribed time intervals.

The light receiving unit 22 has, for example, a photodiode, and detects intensity of light input from the optical fiber 11, that is, intensity of the light passing through the sensor part 11a. The light receiving unit 22 can also be referred to as a detection unit.

The control unit 30 can acquire the intensity of the light received at the light receiving unit 22. In addition, the control unit 30 can switch emission and non-emission of test light at the light source 21 and change the output state of the test light.

The catheter 13 is placed on the surface of the living body A, and its tip (not illustrated) is inserted into a blood vessel V.

The inside of the tube set 12 is filled with a fluid different from blood, such as a saline solution F2, for example. The catheter 13 and the tube set 12 are configured such that the blood pressure inside the blood vessel V is propagated to the saline solution F2. The saline solution F2 is an example of an ex vivo fluid, which may also be referred to as a second fluid.

The tube set 12 includes a tube 12a and a chamber 12b expanded from the tube 12a. The chamber 12b has the sensor part 11a of the optical fiber 11 inserted thereto. As is apparent from FIG. 1, the tube set 12 is located outside the living body A. Therefore, in the present embodiment, the sensor part 11a is located outside the living body A. The sensor part 11a detects the pressure of the saline solution F2 to which the pressure of blood is propagated. Note that the tube set 12 may not have the chamber 12b, and the sensor part 11a may be inserted into the tube 12a.

FIG. 2 is a schematic diagram of the optical fiber 11. As illustrated in FIG. 2, the optical fiber 11 includes the sensor part 11a and two delivery optical fibers 11d. The sensor part 11a is an optical fiber interposed between the two delivery optical fibers 11d. In other words, one delivery optical fiber 11d, the sensor part 11a that is an optical fiber, and the other delivery optical fiber 11d are mechanically and optically connected in series. The transmission loss of the delivery optical fiber 11d is lower than the transmission loss of the sensor part 11a. The effective relative refractive-index difference of the delivery optical fiber 11d is larger than the effective relative refractive-index difference of the sensor part 11a. Furthermore, at a boundary 11f between the sensor part 11a and the delivery optical fiber 11d, the sensor part 11a and the delivery optical fiber 11d are fusion-spliced. The sensor part 11a is an example of a sensor optical fiber.

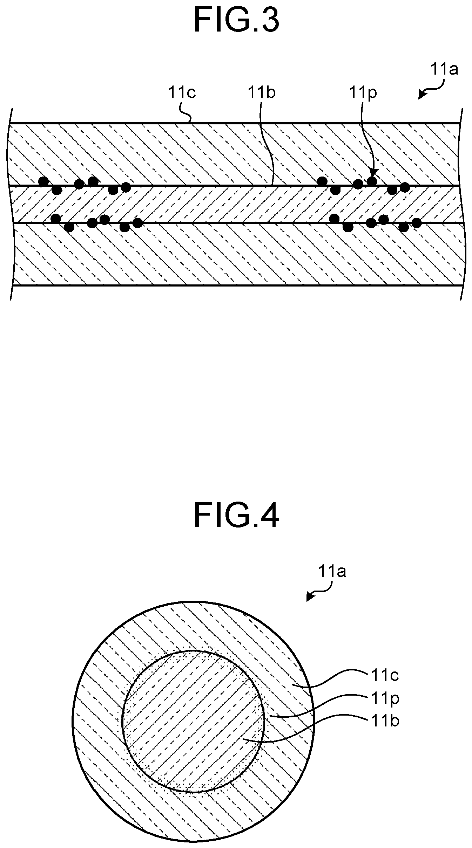

FIG. 3 is a cross-sectional view of a part of the sensor part 11a along the longitudinal direction, and FIG. 4 is a cross-sectional view of the sensor part 11a perpendicular to the longitudinal direction.

As is apparent from FIGS. 3 and 4, the sensor part 11a includes a core 11b and a cladding 11c that surrounds the core 11b and has a lower refractive index than that of the core 11b.

The diameter of the core 11b and the relative refractive-index difference of the core 11b with respect to the cladding 11c are set such that the sensor part 11a can transmit test light in a single mode. Furthermore, the cladding 11c may be surrounded by a coating (not illustrated). The coating in this case exhibits transparency to the test light.

Furthermore, as an example, the diameter of the cladding, that is, the outer diameter of the core wire, is the same for the sensor part 11a and the delivery optical fiber 11d. The diameter (outer diameter) of the core may also be the same or different for the sensor part 11a and the delivery optical fiber 11d. As an example, the diameter of the core of the delivery optical fiber 11d may be larger than the diameter of the core 11b of the sensor part 11a. Furthermore, the delivery optical fiber 11d may be a multi-mode optical fiber that transmits the test light in a multi-mode.

The sensor part 11a and the delivery optical fiber 11d are what is called plastic fibers made of a synthetic resin material transparent to the test light, such as methacrylic resin or fluororesin. However, not limited to this, the sensor part 11a and the delivery optical fiber 11d may be glass optical fibers made of a quartz glass material. The sensor part 11a and the delivery optical fiber 11d may also be made of materials different from each other.

Furthermore, as illustrated in FIGS. 3 and 4, the sensor part 11a includes a plurality of nanostructures 11p near the interface between the core 11b and the cladding 11c. However, distribution of such nanostructures 11p is an example, and the nanostructures 11p may exist throughout the entire radial direction of the cladding 11c within the sensor part 11a. Each of the nanostructures 11p may contain fillers (for example, particles such as fine particles or cylindrical tubes) and voids (for example, microscopic spaces of air other than tubes or fine particles), and may contain at least two kinds of those examples. The nanostructure 11p, for example, has a cross-sectional diameter of 100 [nm] or less in the cross section perpendicular to the longitudinal direction of the sensor part 11a. In this case, the loss in the sensor part 11a tends to increase compared to the case without fillers and
voids. Fillers and voids may be contained more in the cladding 11c than in the core 11b of the sensor part 11a.

Through the intensive sturdies of the inventors, it is found that in the sensor part 11a with such a configuration, the test light is scattered by the nanostructures 11p, which makes it more difficult for the test light to be confined in the core 11b than in a configuration without the nanostructures 11p, that is, the test light tends to leak from the core 11b. In this case, it is also found that the greater the external force acting on the sensor part 11a, the greater the leakage of the test light from the sensor part 11a, that is, the greater the transmission loss in the sensor part 11a. Furthermore, for such a sensor part 11a where the test light tends to leak from the core 11b, it is also found that, when an external force is applied, the transmission loss changes more sensitively in response to the external force. As an example, it is found that such characteristics become more prominent when the transmission loss for the test light at the sensor part 11a is 0.3 [dB/m] or more. This is because light leaks to the outside of the cladding 11c when the transmission loss is 0.3 [dB/m] or more, and light sensitively reacts to changes in the nanostructures 11p near the cladding 11c caused due to the external pressure, which significantly affects the transmission loss.

From these characteristics of the sensor part 11a, the control unit 30 can calculate the pressure of the saline solution F2 at the sensor part 11a corresponding to the intensity of light received at the light receiving unit 22, and thus the pressure of blood, that is, the blood pressure, based on a correlation between the intensity of light received at the light receiving unit 22 acquired in advance experimentally and the pressure acted on the sensor part 11a. The control unit 30 is an example of an arithmetic processing unit.

Furthermore, as illustrated in FIG. 2, the sensor part 11a has a curved part 11a1 folded in a U-like shape. The curved part 11a1 can also be referred to as a bent part or a folded part. Through the intensive studies of the inventors, for the sensor part 11a, it is found that a length L of the sensor part 11a is preferable to be between 1 [cm] and 4 [cm], both inclusive, and a curvature radius R of the curved part 11a1 (radius of the central axis of the sensor part 11a) to be between 50 [µm] and 200 [µm], both inclusive.

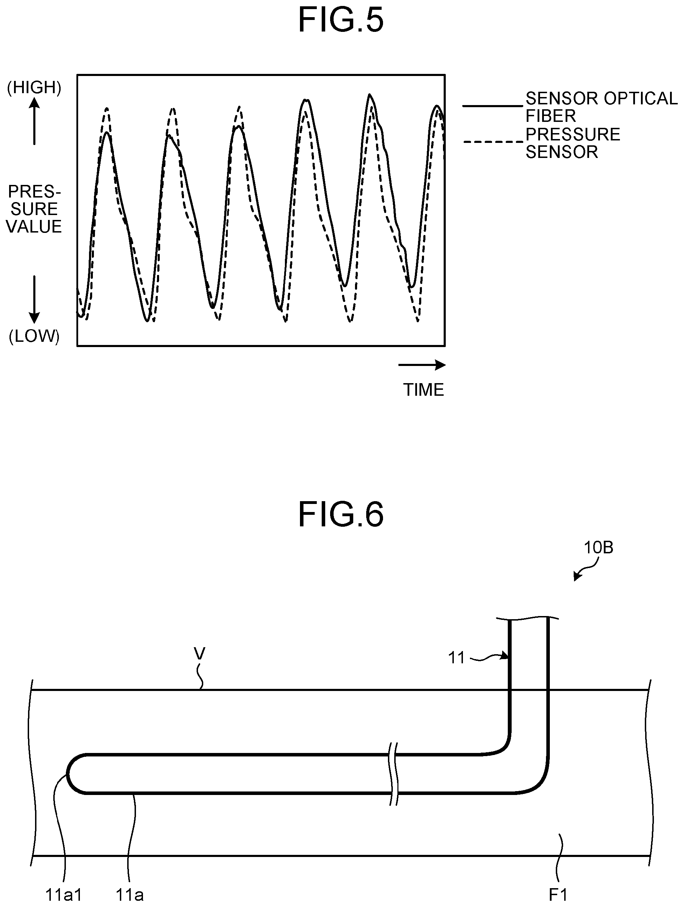

FIG. 5 is a graph presenting an example of changes over time of detection values of pressure acquired by the pressure measurement device 10A of the present embodiment and a conventional pressure sensor. This graph indicates the measurement results of the pressure of a test fluid in an experimental facility that can schematically create pressure changes in the test fluid equivalent to changes in blood pressure caused by heart beats. From FIG. 5, it can be seen that the detection values of the pressure of the test fluid are in good agreement between the pressure measurement device 10A of the present embodiment indicated by a solid line and the conventional pressure sensor indicated by a dashed line. The inventors have experimentally confirmed that, according to the pressure measurement device 10A of the present embodiment, it is possible to acquire the measurement performance that is equal to or better than that of conventional pressure sensors, as in this example.

As described above, in the present embodiment, the light receiving unit 22 receives the test light emitted from the light source 21 and transmitted in the optical fiber 11 including the sensor part 11a (sensor optical fiber). The transmission loss of the test light at the sensor part 11a increases as the external force such as fluid pressure acting on the sensor part 11a increases. The control unit 30 measures the blood pressure (fluid pressure in a living body) acting on the sensor part 11a based on the optical intensity of the test light received at the light receiving unit 22.

With such a configuration, the pressure measurement device 10A can be achieved by a more compact and simpler configuration with optical fibers being the base.

In addition, as in the present embodiment, the sensor part 11a may be a plastic fiber.

With such a configuration, for example, the sensor part 11a can be configured more flexibly, so that the increment ratio of the transmission loss of the test light to the increment in the fluid pressure in the sensor part 11a, that is, the rate of increase, can be made larger. In other words, such a configuration can increase the detection sensitivity of the sensor part 11a for the fluid pressure.

Furthermore, as in the present embodiment, the sensor part 11a may be the optical fiber that transmits the test light with a loss of 0.3 [dB/m] or more.

As in the present embodiment, the sensor part 11a may also have the curved part 11a1.

In addition, as in the present embodiment, the sensor part 11a may include the plurality of nanostructures 11p.

Furthermore, in the present embodiment, the delivery optical fiber 11d between the sensor part 11a and the light source 21 is a multi-mode optical fiber, while the sensor part 11a may be a single-mode optical fiber.

With such a configuration, for example, the test light tends to leak more easily from the sensor part 11a, so that the detection sensitivity of the sensor part 11a for the fluid pressure can be increased.

Furthermore, as in the present embodiment, the light receiving unit 22 may also receive the test light that is input into the optical fiber 11 from the light source 21 via the end part 11e1 (one end) and output from the end part 11e2 (other end) via the sensor part 11a.

With such a configuration, the intensity of received light can be increased, and thus the measurement accuracy can be further improved, compared to a configuration in which the light receiving unit 22 receives light reflected at one end of the optical fiber 11, for example.

Furthermore, in the present embodiment, the sensor part 11a measures the pressure of the saline solution F2 (ex vivo fluid) to which the blood pressure (pressure of an in vivo fluid) is propagated.

The configuration of the present embodiment can be applied to an invasive blood pressure measurement device that indirectly measures the pressure of an in vivo fluid via an ex vivo fluid by the sensor part 11a located outside a living body.

### [Second Embodiment]

FIG. 6 is a schematic diagram of a part of a pressure measurement device 10B according to a second embodiment, which is a cross-sectional view along the longitudinal direction of an optical fiber 11 in a blood vessel V. The optical fiber 11 of the present embodiment is optically connected to a light source 21 and a light receiving unit 22, as in the first embodiment described above. In other words, in the present embodiment, test light input into the optical fiber 11 from the light source 21 is also transmitted in the optical fiber 11 including a sensor part 11a, and received at the light receiving unit 22. A control unit 30 calculates the pressure acted on the sensor part 11a based on the optical intensity of the test light received at the light receiving unit 22.

As illustrated in FIG. 6, in the present embodiment, the optical fiber 11 is inserted into the blood vessel V. With such a configuration, the higher the pressure of blood F1 that is a fluid in the blood vessel V, the greater the transmission loss of the test light at the sensor part 11a. The blood F1 is an example of an in vivo fluid, which may also be referred to as a first fluid.

Through the intensive studies of the inventors, for the sensor part 11a also in the pressure measurement device 10B of the present embodiment, it is found that a length L of the sensor part 11a (see FIG. 2) is preferable to be between 1 [cm] and 4 [cm], both inclusive, and a curvature radius R of a curved part 11a1 (radius of the central axis of the sensor part 11a, see FIG. 2) to be between 50 [µm] and 200 [µm], both inclusive. The area of such a curvature radius R is an area where the bending loss increases sensitively to the pressure, which further improves the sensor sensitivity to the pressure.

With the present embodiment, it is possible to acquire the effects similar to those of the first embodiment described above. The pressure measurement device 10B of the present embodiment can be applied to an invasive blood pressure measurement device that directly measures the pressure of an in vivo fluid by the sensor part 11a located inside a living body.

### [Third Embodiment]

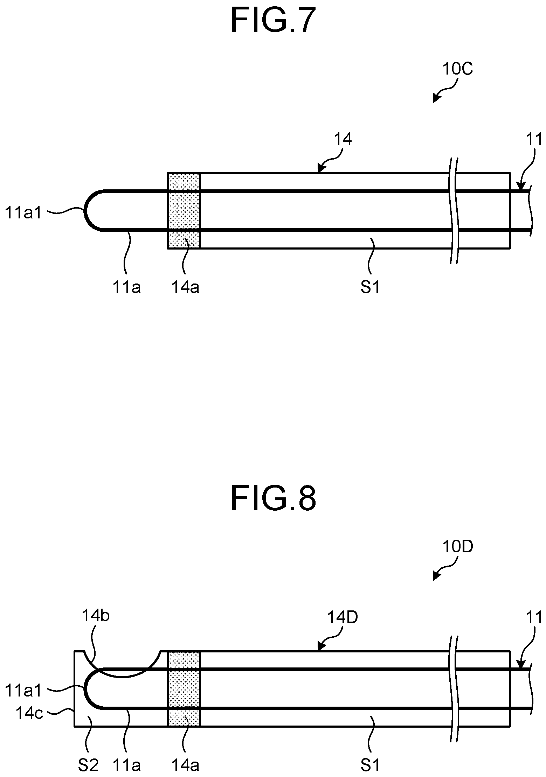

FIG. 7 is a schematic diagram of a part of a pressure measurement device 10C according to a third embodiment, which is a cross-sectional view along the longitudinal direction of an optical fiber 11. The optical fiber 11 of the present embodiment is optically connected to a light source 21 and a light receiving unit 22, as in the first embodiment described above. In other words, in the present embodiment, test light input into the optical fiber 11 from the light source 21 is also transmitted in the optical fiber 11 including a sensor part 11a, and received at the light receiving unit 22. A control unit 30 calculates the pressure acted on the sensor part 11a based on the optical intensity of the test light received at the light receiving unit 22. The sensor part 11a of the present embodiment is inserted into the blood vessel V, as in the second embodiment described above.

As illustrated in FIG. 7, in the present embodiment, the pressure measurement device 10C includes a tubular member 14 that surrounds the optical fiber 11. In other words, the tubular member 14 houses the optical fiber 11 inside the tube. The tubular member 14 surrounds both outward path and return path of the optical fiber 11 to/from the sensor part 11a, and the sensor part 11a is exposed from the tip of the tubular member 14. The tubular member 14 protects the optical fiber 11.

The optical fiber 11 penetrates through a fixing member 14a. The fixing member 14a positions the optical fiber 11 and the tubular member 14 in prescribed relative positions. In the present embodiment, the fixing member 14a, for example, fixes the optical fiber 11 and the tubular member 14 such that the optical fiber 11 is separated from the peripheral wall of the tubular member 14 at least at the position where the fixing member 14a is provided, and that the outward path and the return path of the optical fiber 11 are separated from each other. While the pressure measurement device 10C in the example of FIG. 7 has only one fixing member 14a, the pressure measurement device 10C is not limited thereto and may have a plurality of fixing members 14a disposed with a space provided therebetween along the longitudinal direction of the tubular member 14. The fixing member 14a is an adhesive, for example, but not limited thereto.

Furthermore, the tubular member 14 exhibits flexibility and elasticity. Thus, with such a configuration, by setting the specifications of the tubular member 14 appropriately, for example, the subassembly having the tubular member 14 and the optical fiber 11 can be given moderate elasticity, and thus moderate followability along the blood vessel V. Therefore, it is possible to acquire such an effect that the sensor part 11a is easily inserted into the blood vessel V and also easily removed from the blood vessel V. The tubular member 14 is made of a metallic material or a synthetic resin material, for example. The tubular member 14 is also an example of a support member that exhibits flexibility and supports the optical fiber 11.

With the present embodiment, it is also possible to acquire the effects similar to those of the first embodiment described above. The pressure measurement device 10C of the present embodiment can be applied to an invasive blood pressure measurement device that directly measures the pressure of an in vivo fluid by the sensor part 11a located inside a living body.

### [Modification of Third Embodiment]

FIG. 8 is a schematic diagram of a part of a pressure measurement device 10D according to a modification of the third embodiment, which is a cross-sectional view along the longitudinal direction of an optical fiber 11 in a blood vessel V. The optical fiber 11 of the present modification is optically connected to a light source 21 and a light receiving unit 22, as in the first embodiment described above. In other words, in the present modification, test light input into the optical fiber 11 from the light source 21 is also transmitted in the optical fiber 11 including a sensor part 11a, and received at the light receiving unit 22. A control unit 30 calculates the pressure acted on the sensor part 11a based on the optical intensity of the test light received at the light receiving unit 22. Furthermore, the sensor part 11a of the present modification is inserted into a blood vessel V, as in the third embodiment described above.

As illustrated in FIG. 8, in the present modification, a cover 14c that is a tip part (one part) of a tubular member 14D covers the sensor part 11a. Note, however, that an opening 14b is provided in the peripheral wall (sidewall) of the cover 14c. Thereby, the sensor part 11a comes in contact with the blood F1 that is introduced into the cover 14c from the blood vessel V via the opening 14b. In other words, the sensor part 11a is located in the blood. Furthermore, a fixing member 14a sections the inside of the tube of the tubular member 14 into a space S1 and a space S2 that is opened by the opening 14b. At least a part of the sensor part 11a is disposed in the space S2. With such a configuration, the sensor part 11a along with the optical fiber 11 can be protected by the tubular member 14D. Note that the cover 14c may be configured with a member that is different from the tubular member 14 and attached to the tubular member 14.

The pressure measurement device 10D of the present modification can be applied to an invasive blood pressure measurement device that directly measures the pressure of an in vivo fluid by the sensor part 11a located inside a living body.

### [Fourth Embodiment]

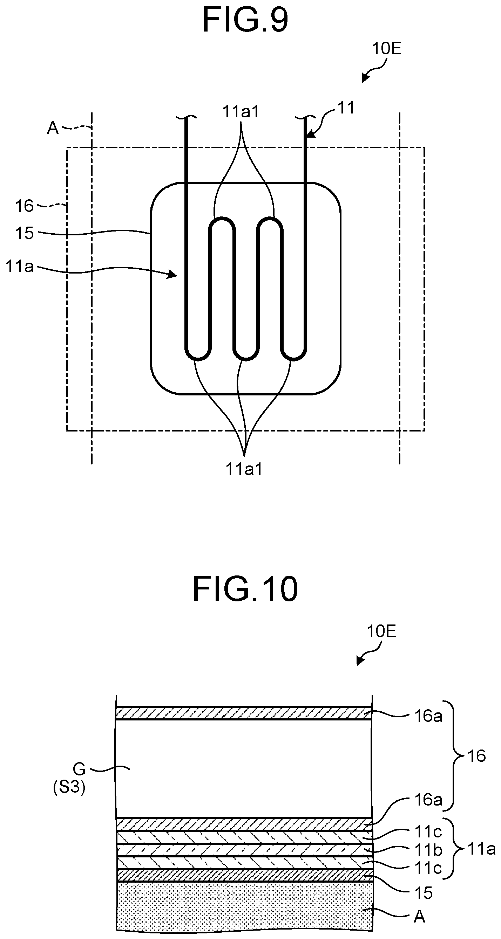

FIG. 9 is a schematic diagram of a pressure measurement device 10E according to a fourth embodiment, which is a plan view illustrating a state being attached outside a living body A. An optical fiber 11 of the present embodiment is optically connected to a light source 21 and a light receiving unit 22, as in the first embodiment described above. In other words, in the present embodiment, test light input into the optical fiber 11 from the light source 21 is also transmitted in the optical fiber 11 including a sensor part 11a, and received at the light receiving unit 22. A control unit 30 calculates the pressure acted on the sensor part 11a based on the optical intensity of the test light received at the light receiving unit 22.

As illustrated in FIG. 9, in the present embodiment, the sensor part 11a is disposed, together with a pad 15 and a cuff 16, on the outer skin of the living body A, such as an arm, for example.

The pad 15 exhibits flexibility and has a sheet-like shape. The pad 15 is placed on the outer skin of the living body A while being in contact therewith. The sensor part 11a (optical fiber 11) is disposed on the opposite side of the outer skin of the living body A with respect to the pad 15. In other words, the pad 15 is interposed between the sensor part 11a and the outer skin of the living body A. The sensor part 11a is fixed to the pad 15 by, for example, an adhesive or the like.

The cuff 16 is wrapped around the living body A, for example, around the arm, so as to cover the pad 15 and the sensor part 11a.

Furthermore, the sensor part 11a is bent a plurality of times via a plurality of curved parts 11a1. Such a configuration can expand the area where sensing is possible.

FIG. 10 is a cross-sectional view of the pressure measurement device 10E perpendicular to the pad 15. As illustrated in FIG. 10, the pad 15, the sensor part 11a, and the cuff 16 are stacked in this order on the outer skin of the living body A. The sensor part 11a is disposed between the pad 15 and the cuff 16. The pad 15, the sensor part 11a, and the cuff 16 may or may not be fixed to each other by an adhesive or the like.

The cuff 16 has a space S3 capable of housing gas G such as air, for example, provided between a sheet 16a close to the living body A and a sheet 16a far from the living body A. The space S3 is connected to an electric pump and an electric valve, not illustrated. The cuff 16 is configured such that the pressure of the gas G in the space S3 can be changed by controlling the electric pump and the electric valve. Specifically, the cuff 16 is configured to allow the pressure of the gas G in the space S3 to increase and then to decrease gradually over time, for example. With such a configuration, the control unit 30 can acquire the maximum blood pressure and the minimum blood pressure by well-known oscillometric methods in blood pressure monitors using the cuff 16, for example, based on the detection values of the sensor part 11a or the changes over time of the blood pressure pulsation acquired from the detection values.

With the present embodiment, it is also possible to acquire the effects similar to those of the first embodiment described above. The pressure measurement device 10E of the present embodiment can be applied to a non-invasive blood pressure measurement device that indirectly measures the pressure of an in vivo fluid via the living body A by the sensor part 11a located outside the living body.

### [Fifth Embodiment]

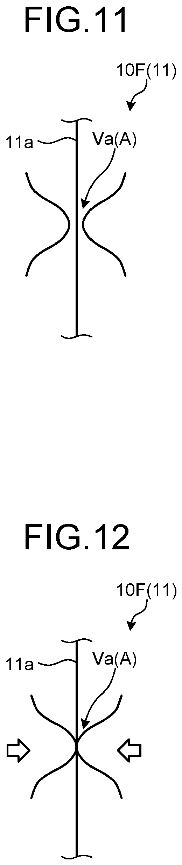

FIGS. 11 and 12 are schematic diagrams of a pressure measurement device 10F according to a fifth embodiment. The pressure measurement device 10F measures not the pressure of a fluid in the living body A but the pressure acting on a sensor part 11a of an optical fiber 11 from a valve part Va of the living body A, that is, the pressure received from a body part in the living body. The valve part Va has a constriction part (throat), and the sensor part 11a penetrates through the constriction. The valve part Va is, for example, the heart valve, the pylorus of the stomach, or the like. FIG. 11 illustrates a state where the constriction part is widened relatively large, with no pressure acting on the sensor part 11a from the valve part Va, that is, from the living body A. On the other hand, FIG. 12 illustrates a state where the constriction part is narrowed relatively small, with pressure acting on the sensor part 11a from the valve part Va. In the state illustrated in FIG. 12, the outer periphery of the sensor part 11a is dented due to the force received from the valve part Va, thereby increasing leakage of the test light from the sensor part 11a and increasing the transmission loss for the test light at the sensor part 11a compared to the state illustrated in FIG. 11. Therefore, the control unit 30 can calculate the pressure acted on the sensor part 11a corresponding to the intensity of light received at the light receiving unit 22 based on a correlation between the intensity of the light received at the light receiving unit 22 acquired in advance experimentally and the pressure acted on the sensor part 11a from the valve part Va. Note that the pressure measurement device 10F can also measure the force (load) acted on the sensor part 11a from the valve part Va. In other words, in the present embodiment, the pressure measurement device 10F can also function as an in vivo force measurement device (load measurement device).

### [Modification of Sensor Part]

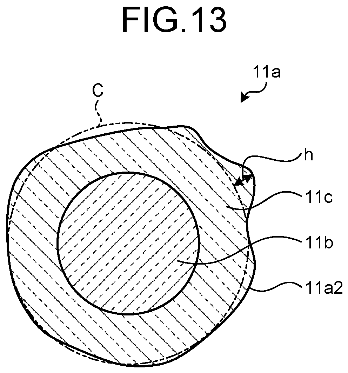

FIG. 13 is a cross-sectional view perpendicular to the longitudinal direction of a sensor part 11a according to a modification of the embodiments. As illustrated in FIG. 13, an outer periphery 11a2 of the sensor part 11a, that is, an outer periphery 11a2 of a cladding 11c, may be provided with an uneven structure including concave or convex parts. The uneven structure can also be referred to as structural fluctuation. By setting the size of the uneven structure, such as the length of the uneven structure in the longitudinal direction, the spacing, and the height difference in the radial direction, for example, to appropriate values, the transmission loss for the test light at the sensor part 11a can be made to 0.3 dB/m or more. Through the intensive studies of the inventors, it is found that the size of the concave and convex of the uneven structure is preferable to be between 5 [nm] and 100 [nm], both inclusive. As an example of an index of the size of the uneven structure, FIG. 13 illustrates a deviation h of the average radius of the outer periphery 11a2 from a circumference C in the cross section perpendicular to the longitudinal direction of the sensor part 11a. The deviation h is also preferable to be between 5 [nm] and 100 [nm], both inclusive. Note that the uneven structure may be provided on the outer periphery of the core 11b. In addition, in the present modification, the sensor part 11a can also be made of plastic fibers, for example.

For example, the configurations of the embodiments and modifications described above can also be applied to measurement of pressure of in vivo fluids other than blood, measurement of pressure received from body parts in a living body other than the valve part such as the heart valve, the pylorus of the stomach, and the like.

Furthermore, the support member is not limited to the tubular member, but may be a rod-shaped member or the like that exhibits flexibility, for example. In that case, the optical fiber may extend linearly or spirally along the outer periphery of the support member. Furthermore, the optical fiber may be housed in a linear or helical groove provided on the outer periphery of the support member.

In addition, optical fibers with different mode transmission states from each other may be applied to the sensor part and the delivery optical fiber such that mode conversion occurs at the connection point (boundary)
between the sensor part (sensor optical fiber) and the delivery optical fiber between the sensor part and the light source. In this case, mode conversion at the connection point depends on the external force, and the mode mismatch state also depends on the external force. Therefore, the degree of leakage of the test light at the sensor part becomes more sensitive to the external force, which results in increasing the sensitivity of the sensor part further to the pressure in the living body.

### Industrial Applicability

The present invention can be utilized for in vivo pressure measurement devices.

### Reference Signs List

10A to 10F Pressure measurement device (in vivo pressure measurement device)
11 Optical fiber
11a Sensor part (sensor optical fiber)
11a1 Curved part
11a2 Outer periphery
11b Core
11c Cladding
11d Delivery optical fiber
11e1 End part (one end)
11e2 End part (other end)
11f Boundary
11p Nanostructure
12 Tube set
12a Tube
12b Chamber
13 Catheter
14, 14D Tubular member (support member)
14a Fixing member
14b Opening
14c Cover
15 Pad
16 Cuff
16a Sheet
21 Light source
22 Light receiving unit
30 Control unit (arithmetic processing unit)
A Living body
C Circumference
F1 Blood (in vivo fluid)
F2 Saline solution (ex vivo fluid)
G Gas
h Deviation
L Length
R Curvature radius
S1 to S3 Space
V Blood vessel
Va Valve part

## Claims

1. An in vivo pressure measurement device comprising:
a light source (21) configured to output test light;
an optical fiber (11), to which the test light is input, at least partially including a sensor optical fiber (11a) configured to transmit the test light with a loss of 0.3 dB/m or more, wherein the sensor optical fiber (11a) includes
a core (11b), and
a cladding (11c) surrounding the core (11b); and
a light receiving unit (22) configured to receive the test light transmitted through the sensor optical fiber (11a), wherein
the in vivo pressure measurement device is configured to measure pressure in a living body acting on the sensor optical fiber (11a) based on intensity of the test light received at the light receiving unit (22),
**characterized in that**
the sensor optical fiber (11a) includes a plurality of nanostructures (11p) near an interface between the core (11b) and the cladding (11c), and
the nanostructure (11p) has a cross-sectional diameter of 100 nm or less in a cross section perpendicular to a longitudinal direction of the sensor optical fiber (11a).

2. The in vivo pressure measurement device according to claim 1, wherein the pressure in the living body is pressure of blood in the living body.

3. The in vivo pressure measurement device according to claim 1 or 2, wherein the sensor optical fiber (11a) is a plastic fiber.

4. The in vivo pressure measurement device according to any one of claims 1 to 3, wherein the sensor optical fiber (11a) includes a curved part (11a1).

5. The in vivo pressure measurement device according to any one of claims 1 to 4, wherein the sensor optical fiber (11a) is configured to be inserted into the living body.

6. The in vivo pressure measurement device according to any one of claims 1 to 5, comprising a flexible support member (14, 14D) configured to extend along the optical fiber (11) and support the optical fiber (11).

7. The in vivo pressure measurement device according to any one of claims 1 to 6, comprising a tubular member configured to house the optical fiber (11a).

8. The in vivo pressure measurement device according to claim 7, wherein the tubular member is flexible.

9. The in vivo pressure measurement device according to any one of claims 1 to 8, comprising a cover configured to cover the sensor optical fiber (11a) in a state capable of introducing an in vivo fluid or an ex vivo fluid (F2) to which pressure of the in vivo fluid is propagated.

10. The in vivo pressure measurement device according to any one of claims 1 to 4, wherein the sensor optical fiber (11a) is configured to be located outside the living body, and configured to measure pressure of an in vivo fluid indirectly acting on the sensor optical fiber via the living body.

11. The in vivo pressure measurement device according to claim 10, wherein the sensor optical fiber (11a) is configured to be disposed between the living body and a cuff located outside the living body, the cuff being configured to constrict a blood vessel in the living body by internal air pressure and change the air pressure.

12. The in vivo pressure measurement device according to any one of claims 1 to 11, wherein the optical fiber includes:
the sensor optical fiber; and
a delivery optical fiber (11d) connected to the sensor optical fiber (11a) and having a lower transmission loss than the sensor optical fiber (11a) does.

13. The in vivo pressure measurement device according to claim 12, wherein, in wavelengths of the test light,
the sensor optical fiber (11a) is a single-mode optical fiber, and
the delivery optical fiber (11d) between the sensor optical fiber and the light source (21) is a multi-mode optical fiber.

14. The in vivo pressure measurement device according to claim 12 or 13, wherein the sensor optical fiber (11a) and the delivery optical fiber (11d) are fusion-spliced.

15. The in vivo pressure measurement device according to any one of claims 1 to 14, wherein the light receiving unit (22) is configured to receive the test light input from one end of the optical fiber (11) and output from another end of the optical fiber (11).

16. The in vivo pressure measurement device according to any one of claims 1 to 15, wherein
the sensor optical fiber is configured to measure the pressure in an in vivo fluid (F1) by:
measuring pressure of the in vivo fluid (F1); or
measuring pressure of an ex vivo fluid (F2) to which pressure of the in vivo fluid is propagated.

17. The in vivo pressure measurement device according to claim 16, wherein the nanostructure (11p) is a microparticle, a tube or a void.

18. The in vivo pressure measurement device according to any one of claims 1 to 17, wherein an outer periphery of the sensor optical fiber (11a) includes an uneven structure of 5 nm to 100 nm.

19. The in vivo pressure measurement device according to any one of claims 1 to 18, comprising an arithmetic processing unit (30) configured to acquire the pressure acting on the sensor optical fiber (11a) based on the intensity of the test light.

## Patentansprüche

1. In-vivo-Druckmessvorrichtung, umfassend:
eine Lichtquelle (21), die so ausgelegt ist, dass sie Testlicht aussendet;
eine optische Faser (11), in die das Testlicht eingekoppelt wird und die zumindest teilweise eine Sensor-Lichtleitfaser (11a) umfasst und die so ausgelegt ist, dass sie das Testlicht mit einem Verlust von 0,3 dB/m oder mehr überträgt, wobei die Sensor-Lichtleitfaser (11a) umfasst
einen Kern (11b) und
einen Mantel (11c), der den Kern (11b) umgibt; und
eine Lichtempfangseinheit (22), die so ausgelegt ist, dass sie das durch die Sensor-Lichtleitfaser (11a) übertragene Testlicht empfängt, wobei
die In-vivo-Druckmessvorrichtung so ausgelegt ist, dass sie den auf die Sensor-Lichtleitfaser (11a) einwirkenden Druck in einem lebenden Körper auf der Grundlage der Intensität des an der Lichtempfangseinheit (22) empfangenen Testlichts misst,
**dadurch gekennzeichnet, dass**
die Sensor-Lichtleitfaser (11a) eine Vielzahl von Nanostrukturen (11p) in der Nähe einer Grenzfläche zwischen dem Kern (11b) und dem Mantel (11c) aufweist, und
die Nanostruktur (11p) in einem Querschnitt senkrecht zur Längsrichtung der Sensor-Lichtleitfaser (11a) einen Querschnittsdurchmesser von 100 nm oder weniger aufweist.

2. In-vivo-Druckmessvorrichtung nach Anspruch 1, wobei der Druck im lebenden Körper der Blutdruck im lebenden Körper ist.

3. In-vivo-Druckmessvorrichtung nach Anspruch 1 oder 2, wobei die Sensor-Lichtleitfaser (11a) eine Kunststofffaser ist.

4. In-vivo-Druckmessvorrichtung nach einem der Ansprüche 1 bis 3, wobei die Sensor-Lichtleitfaser (11a) einen gekrümmten Abschnitt (11a1) aufweist.

5. In-vivo-Druckmessvorrichtung nach einem der Ansprüche 1 bis 4, wobei die Sensor-Lichtleitfaser (11a) so ausgebildet ist, dass sie in den lebenden Körper eingeführt werden kann.

6. In-vivo-Druckmessvorrichtung nach einem der Ansprüche 1 bis 5, umfassend ein flexibles Stützelement (14, 14D), das so ausgebildet ist, dass es sich entlang der Lichtleitfaser (11) erstreckt und die Lichtleitfaser (11) stützt.

7. In-vivo-Druckmessvorrichtung nach einem der Ansprüche 1 bis 6, umfassend ein röhrenförmiges Element, das so ausgebildet ist, dass es die optische Faser (11a) aufnimmt.

8. In-vivo-Druckmessvorrichtung nach Anspruch 7, wobei das röhrenförmige Element flexibel ist.

9. In-vivo-Druckmessvorrichtung nach einem der Ansprüche 1 bis 8, umfassend eine Abdeckung, die so ausgebildet ist, dass sie die optische Sensorfaser (11a) in einem Zustand abdeckt, in dem eine In-vivo-Flüssigkeit oder eine Ex-vivo-Flüssigkeit (F2) zugeführt werden kann, auf die der Druck der In-vivo-Flüssigkeit übertragen wird.

10. In-vivo-Druckmessvorrichtung nach einem der Ansprüche 1 bis 4, wobei die Sensor-Lichtleitfaser (11a) so ausgebildet ist, dass sie sich außerhalb des lebenden Körpers befindet, und so ausgebildet ist, dass sie den Druck einer In-vivo-Flüssigkeit misst, der über den lebenden Körper indirekt auf die Sensor-Lichtleitfaser einwirkt.

11. In-vivo-Druckmessvorrichtung gemäß Anspruch 10, wobei die optische Sensorfaser (11a) so ausgebildet ist, dass sie zwischen dem lebenden Körper und einer außerhalb des lebenden Körpers angeordneten Manschette angeordnet ist, wobei die Manschette so ausgebildet ist, dass sie ein Blutgefäß im lebenden Körper durch inneren Luftdruck verengt und den Luftdruck verändert.

12. In-vivo-Druckmessvorrichtung nach einem der Ansprüche 1 bis 11, wobei die optische Faser umfasst:
die Sensor-Lichtleitfaser; und
eine mit der Sensor-Lichtleitfaser (11a) verbundene Übertragungs-Lichtleitfaser (11d), die einen geringeren Übertragungsverlust aufweist als die Sensor-Lichtleitfaser (11a).

13. In-vivo-Druckmessvorrichtung nach Anspruch 12, wobei bei den Wellenlängen des Testlichts
die Sensor-Lichtleitfaser (11a) eine Einmoden-Lichtleitfaser ist und
die Zuführungslichtleitfaser (11d) zwischen der Sensor-Lichtleitfaser und der Lichtquelle (21) eine Multimode-Lichtleitfaser ist.

14. In-vivo-Druckmessvorrichtung nach Anspruch 12 oder 13, wobei die Sensor-Lichtleitfaser (11a) und die Zuführungs-Lichtleitfaser (11d) durch Fusionsspleißen verbunden sind.

15. In-vivo-Druckmessvorrichtung nach einem der Ansprüche 1 bis 14, wobei die Lichtempfangseinheit (22) so konfiguriert ist, dass sie das Testlicht empfängt, das an einem Ende der Lichtleitfaser (11) eingegeben und am anderen Ende der Lichtleitfaser (11) ausgegeben wird.

16. In-vivo-Druckmessvorrichtung nach einem der Ansprüche 1 bis 15, wobei
die Sensor-Lichtleitfaser so ausgelegt ist, dass sie den Druck in einer In-vivo-Flüssigkeit (F1) misst, indem sie:
den Druck der In-vivo-Flüssigkeit (F1) zu messen; oder
Messen des Drucks einer Ex-vivo-Flüssigkeit (F2), auf die sich der Druck der In-vivo-Flüssigkeit ausbreitet.

17. In-vivo-Druckmessvorrichtung nach Anspruch 16, wobei die Nanostruktur (11p) ein Mikropartikel, ein Röhrchen oder ein Hohlraum ist.

18. In-vivo-Druckmessvorrichtung nach einem der Ansprüche 1 bis 17, wobei ein Außenumfang der Sensor-Lichtleitfaser (11a) eine unebene Struktur von 5 nm bis 100 nm aufweist.

19. In-vivo-Druckmessvorrichtung nach einem der Ansprüche 1 bis 18, umfassend eine Recheneinheit (30), die so konfiguriert ist, dass sie den auf die Sensor-Lichtleitfaser (11a) wirkenden Druck auf der Grundlage der Intensität des Testlichts erfasst.

## Revendications

1. Dispositif de mesure de pression in vivo comprenant :
une source de lumière (21) conçue pour émettre une lumière d'essai,
une fibre optique (11), dans laquelle est introduite la lumière d'essai et comprenant au moins en partie une fibre optique de détection (11a) conçue pour transmettre la lumière d'essai avec une perte d'au moins 0,3 dB/m, la fibre optique de détection (11a) comprenant
un noyau (11b), et
une gaine (11c) entourant le noyau (11b), et
une unité de réception de lumière (22) conçue pour recevoir la lumière d'essai transmise à travers la fibre optique de détection (11a) ;
le dispositif de mesure de pression in vivo étant conçu pour mesurer, en fonction de l'intensité de la lumière d'essai reçue par l'unité de réception de lumière (22), la pression agissant, dans un corps vivant, sur la fibre optique de détection (11a) ;
**caractérisé en ce que**
la fibre optique de détection (11a) comprend une pluralité de nanostructures (11p) à proximité d'une interface entre le noyau (11b) et la gaine (11c), et
la nanostructure (11p) présente un diamètre ne dépassant pas 100 nm dans une section transversale perpendiculaire à la direction longitudinale de la fibre optique de détection (11a).

2. Dispositif de mesure de pression in vivo selon la revendication 1, dans lequel la pression dans le corps vivant est la pression du sang dans le corps vivant.

3. Dispositif de mesure de pression in vivo selon la revendication 1 ou 2, dans lequel la fibre optique de détection (11a) est une fibre plastique.

4. Dispositif de mesure de pression in vivo selon l'une quelconque des revendications 1 à 3, dans lequel la fibre optique de détection (11a) comprend une partie courbée (11a1).

5. Dispositif de mesure de pression in vivo selon l'une quelconque des revendications 1 à 4, dans lequel la fibre optique de détection (11a) est conçue pour être insérée dans le corps vivant.

6. Dispositif de mesure de pression in vivo selon l'une quelconque des revendications 1 à 5, comprenant un élément de support souple (14, 14D) conçu pour s'étendre le long de la fibre optique (11) et supporter la fibre optique (11).

7. Dispositif de mesure de pression in vivo selon l'une quelconque des revendications 1 à 6, comprenant un élément tubulaire conçu pour loger la fibre optique (11a).

8. Dispositif de mesure de pression in vivo selon la revendication 7, dans lequel l'élément tubulaire est souple.

9. Dispositif de mesure de pression in vivo selon l'une quelconque des revendications 1 à 8, comprenant un capuchon conçu pour couvrir la fibre optique de détection (11a) dans un état permettant l'introduction d'un fluide in vivo ou d'un fluide ex vivo (F2) auquel est communiquée la pression du fluide in vivo.

10. Dispositif de mesure de pression in vivo selon l'une quelconque des revendications 1 à 4, dans lequel la fibre optique de détection (11a) est conçue pour être située en dehors du corps vivant, et conçue pour mesurer la pression d'un fluide in vivo agissant indirectement sur la fibre optique de détection par le biais du corps vivant.

11. Dispositif de mesure de pression in vivo selon la revendication 10, dans lequel la fibre optique de détection (11a) est conçue pour être disposée entre le corps vivant et un brassard situé en dehors du corps vivant, le brassard étant conçu pour comprimer un vaisseau sanguin du corps vivant par pression d'air interne et pour modifier la pression d'air.

12. Dispositif de mesure de pression in vivo selon l'une quelconque des revendications 1 à 11, dans lequel la fibre optique comprend :
la fibre optique de détection, et
une fibre optique d'application (11d) reliée à la fibre optique de détection (11a) et présentant une perte de transmission plus faible que celle de la fibre optique de détection (11a).

13. Dispositif de mesure de pression in vivo selon la revendication 12, dans lequel, pour les longueurs d'onde de la lumière d'essai :
la fibre optique de détection (11a) est une fibre optique monomodale, et
la fibre optique d'application (11d), entre la fibre optique de détection et la source de lumière (21), est une fibre optique multimodale.

14. Dispositif de mesure de pression in vivo selon la revendication 12 ou 13, dans lequel la fibre optique de détection (11a) et la fibre optique d'application (11d) sont épissées par fusion.

15. Dispositif de mesure de pression in vivo selon l'une quelconque des revendications 1 à 14, dans lequel l'unité de réception de lumière (22) est conçue pour recevoir la lumière d'essai introduite par une extrémité de la fibre optique (11) et émise par l'autre extrémité de la fibre optique (11).

16. Dispositif de mesure de pression in vivo selon l'une quelconque des revendications 1 à 15, dans lequel
la fibre optique de détection est conçue pour mesurer la pression dans un fluide in vivo (F1) par :
mesure de la pression dans le fluide in vivo (F1), ou
mesure de la pression d'un fluide ex vivo (F2) auquel est communiquée la pression du fluide in vivo.

17. Dispositif de mesure de pression in vivo selon la revendication 16, dans lequel la nanostructure (11p) est une microparticule, un tube ou un vide.

18. Dispositif de mesure de pression in vivo selon l'une quelconque des revendications 1 à 17, dans lequel une périphérie extérieure de la fibre optique de détection (11a) comprend une structure irrégulière de 5 nm à 100 nm.

19. Dispositif de mesure de pression in vivo selon l'une quelconque des revendications 1 à 18, comprenant une unité de traitement arithmétique (30) conçue pour obtenir la pression agissant sur la fibre optique de détection (11a) en fonction de l'intensité de la lumière d'essai.
